# EUROPEAN PATENT SPECIFICATION

(11) **EP 2 742 451 B1**
(45) Date of publication and mention of the grant of the patent: **06.11.2019**
(21) Application number: 12772519.0
(22) Date of filing: 14.09.2012
(51) Int. Cl.: G16H 50/20, G16H 20/30

(54) **LIFE RHYTHM PROCESSING SYSTEM FOR RECOMMENDING ACTIVITIES**
LEBENSRHYTHMUSVERARBEITUNGSSYSTEM ZUM VORSCHLAGEN VON AKTIVITÄTEN
SYSTÈME D'ANALYSE DU RYTHME DE VIE POUR RECOMMANDER DES ACTIVITÉS

(30) Priority: 27.09.2011 JP 2011211228
(43) Date of publication of application: 18.06.2014
(73) Proprietor: Sony Corporation, Tokyo 108-0075 (JP)
(72) Inventor: SAKAMOTO, Takayuki, Tokyo 108-0075 (JP); TAKAGI, Tsuyoshi, Tokyo 108-0075 (JP); IKENOUE, Shoichi, Tokyo 108-0075 (JP)
(74) Representative: D Young & Co LLP
(86) International application number: PCT/JP2012/005890
(87) International publication number: WO 2013/046588

(56) References cited:
- WO-A1-00/52604
- WO-A2-2006/079942
- WO-A2-2008/001366
- GB-A- 2 454 705
- US-A- 5 673 691
- US-A1- 2006 183 980
- US-A1- 2008 086 318
- US-B2- 7 261 690

## Description

### TECHNICAL FIELD

The present technique relates to a terminal device, an external device, an information processing method, a program, and an information processing system. Particularly, it is directed for using information corresponding to a user's life rhythm easily and timely.

### BACKGROUND ART

Conventionally, there have been served health care services for providing health care information depending on a user with an information processing system. For example, a terminal device having a communication function and a measurement device measuring vital data (such as body weight, blood pressure and steps) are linked with each other to record client's vital data measured by the measurement device in an external device by the terminal device. The external device performs a statistical processing and the like on the recorded vital data, and transmits a statistical processing result to the terminal device.

In a health care management system described in Patent Document 1, there is proposed, as other health care service, that advice about diet and exercise is given based on life style data and vital data.

### CITATION LIST

### PATENT DOCUMENT

Patent Document 1: Japanese Patent Application Laid-Open No. 11-47096

Other previously proposed arrangements are disclosed in W 2008/001366, US 2006/183980, and US 5673691.

### SUMMARY

### PROBLEMS TO BE SOLVED

With a service for notifying a statistical processing result of vital data, a change in accumulated vital data can be grasped. However, if a daily change in vital data is small, a change in statistical processing result to be notified is also small, and a degree of user's interest can lower and the service can be less used. For information provision, when information is provided through linkage with a vital data registration timing, the user cannot timely obtain the information based on his/her life rhythm.

When a search type system is used so as to provide information a user desires, the user cannot obtain information without a setting operation on search conditions and the like. Thus, if the user determines that the operation is complicated, it is difficult to increase a service use frequency and to cause the user to continuously utilize the service.

It is therefore an object of the present technique to enable information corresponding to a user's life rhythm to be easily and timely used.

### Solution to Problem

The present invention discloses a system, a method and a computer program as defined by claims 1-11.

The scope of the invention is defined only by the claims. Some embodiments described below comprise features which are not part of the claims, thus these embodiments are not covered by the claims.

According to one example, the disclosure is directed to an information processing system comprising: a sensor unit that collects information used for analyzing a life rhythm of a user; a life rhythm analyzing unit that analyzes the user's life rhythm based on the collected information; and a content transmitting unit that acquires an analysis result of the life rhythm of the user from the life rhythm analyzing unit and provides content based on the analysis result.

The sensor unit may be a movement sensor that collects information corresponding to movement of the user, a pedometer that collects information corresponding to a number of steps taken by the user, and/or a location detecting unit that collects information corresponding to a location of the user.

The life rhythm analyzing unit may include a memory that stores the information collected by the sensor unit, and the life rhythm analyzing unit may analyze the user's life rhythm based on the information stored in the memory. The life rhythm analyzing unit may also analyze the user's life rhythm for individual days based on the collected information for each of the individual days stored in the memory.

The information processing system may further include a content registering unit that stores information associating each of a plurality of life rhythm analysis results and at least one piece of content, wherein the content transmitting unit may acquire the content to be provided based on a comparison between the analysis result and the information stored in the content registering unit.

The analysis result may indicate at least one of a pattern of movement and a pattern of exercise corresponding to the user, and the content transmitting unit may acquire the content to be provided based on the at least of the pattern of movement and pattern of exercise corresponding to the user.

The information processing system may further include an environmental information acquiring unit that acquires environmental information corresponding to the user. The environmental information acquiring unit may include a memory that stores the acquired environmental information corresponding to the user. The acquired environmental information may include at least one of weather information, daylight hours, precipitation, wind direction and wind speed. The content transmitting unit may acquire the content to be provided based on the analysis result and the acquired environmental information corresponding to the user.

The information processing system may be configured to include a mobile terminal that includes the sensor unit and the life rhythm analyzing unit; and a server that includes the content transmitting unit, wherein the mobile terminal further includes an interface that sends the analysis result to the server and receives the provided content from the server.

The information processing system may be configured to include a mobile terminal that includes the sensor unit and an interface that sends the collected information to a server; and the server that includes the life rhythm analyzing unit and the content transmitting unit and an interface that sends the provided content to the mobile terminal.

According to another example, the disclosure is directed to an information processing method performed by an information processing system, the method comprising: collecting, by a sensor unit, information used for analyzing a life rhythm of a user; analyzing the user's life rhythm based on the collected information; acquiring an analysis result of the life rhythm based on the analyzing; and providing content based on the analysis result.

According to another example, the disclosure is directed to a non-transitory computer readable medium including computer program instructions, which when executed by an information processing system, causes the information processing system to perform a method comprising: collecting, by a sensor unit, information used for analyzing a life rhythm of a user; analyzing the user's life rhythm based on the collected information; acquiring an analysis result of the life rhythm based on the analyzing; and providing content based on the analysis result.

### Advantageous Effects of Invention

With the technique, in the terminal device, information for selecting content according to a life rhythm is collected and the collected information is provided and a content request is made to the external device. Content selected and supplied according to a life rhythm is presented based on the collected information in the external device in response to the content request. In the external device, content is selected based on the collected information for selecting content according to a life rhythm supplied from the terminal device in response to the content request from the terminal device and the selected content is transmitted to the terminal device. Thus, the information corresponding to the user's life rhythm can be easily and timely used.

### Brief Description of Drawings

Fig. 1 is a block diagram showing a structure of an information processing system.
Fig. 2 is a diagram exemplifying an outer profile of a terminal device.
Fig. 3 is a diagram exemplifying an information input screen.
Fig. 4 is a diagram exemplifying a channel selection screen.
Fig. 5 is a flowchart showing operations of the terminal device.
Fig. 6 is a diagram exemplifying information stored in a collected information storing unit.
Fig. 7 is a diagram exemplifying an action pattern analysis result.
Fig. 8 is a diagram exemplifying an exercise quantity pattern analysis result.
Fig. 9 is a diagram exemplifying a preference pattern analysis result.
Fig. 10 is a diagram exemplifying a day-based pattern analysis result.
Fig. 11 is a flowchart showing operations of a life rhythm analyzing unit.
Fig. 12 is a diagram exemplifying stored content information.
Fig. 13 is a diagram exemplifying stored environmental information.
Fig. 14 is a diagram for explaining action determining operations.
Fig. 15 is a diagram for explaining item determining operations.
Fig. 16 is a diagram for explaining target standard determining operations.
Fig. 17 is a flowchart showing operations of a content transmitting unit.
Fig. 18 is a diagram showing content presenting operations.

### Description of Embodiments

An example of the present technique will be described below. The explanation will be made in the following order.
1. Structure of information processing system
2. Operations of terminal device
3. Operations of life rhythm analyzing unit
4. Operations of server device

### <1. Structure of information processing system>

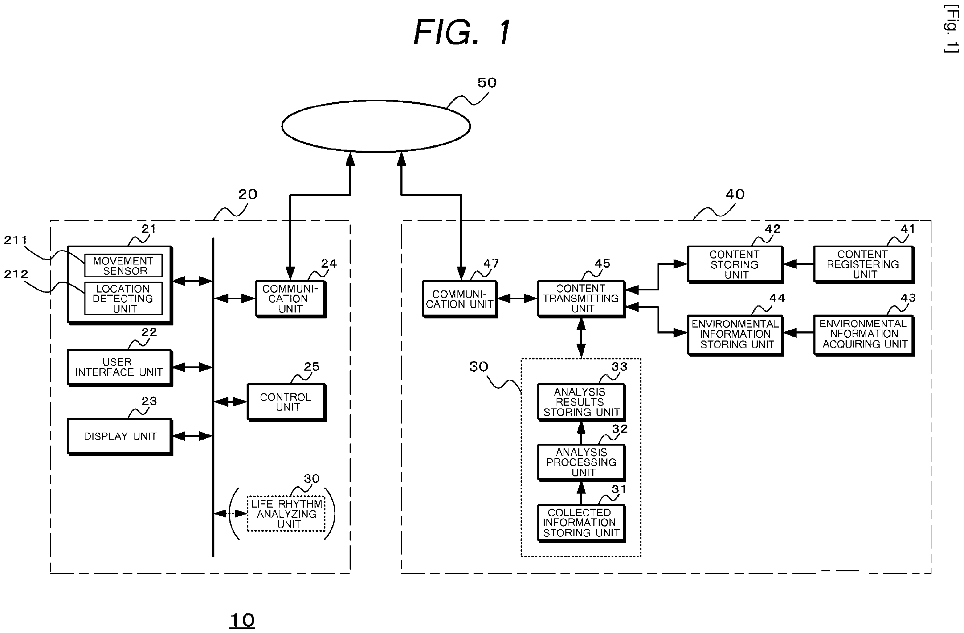

Fig. 1 is a block diagram showing a structure of an information processing system. The information processing system 10 is configured of a terminal device 20 and an external device such as a server device 40, and the terminal device 20 and the server device 40 are connected to each other via a network 50, for example.

The terminal device 20 has a sensor unit 21, a user interface unit 22, a display unit 23, a communication unit 24 and a control unit 25. The server device 40 has a content registering unit 41, a content storing unit 42, an environmental information acquiring unit 43, an environmental information storing unit 44, a content transmitting unit 45, and a communication unit 47. Further, in the information processing system 10, a life rhythm analyzing unit 30 is provided in the terminal device 20 or the server device 40. Fig. 1 exemplifies a case in which the life rhythm analyzing unit is provided in the server device 40. The content is information such as image, character, speech or motor control signal, and the following description exemplifies a case in which the content is information such as image or character.

The sensor unit 21 in the terminal device 20 collects information used for analyzing a life rhythm. For example, the sensor unit 21 is provided with a movement sensor 211 having a step counting function, and generates information on user's motions. The sensor unit 21 may be provided with a location detecting unit 212 obtaining position information. The location detecting unit 212 is provided with a function of receiving a positioning signal used for GPS (Global Positioning System), for example, and detecting a current position, a function of receiving a signal from a base station of cell phones and detecting a current position, a function of obtaining position information of a router based on an address contained in a signal received from the router in WiFi (wireless fidelity) and detecting a current position, and the like. The information collected in the sensor unit 21 and the information input via a user's information input operation described later are assumed as collected information.

The user interface unit 22 is directed for user's menu selection, information input and the like, and is configured of operating switches, a touch panel or the like. The user interface unit 22 generates an operation signal depending on a user operation and outputs the signal to the control unit 25.

The display unit 23 is configured of a flat display device such as liquid crystal device or organic EL. The display unit 23 displays menus or content supplied from the server device 40.

The communication unit 24 makes communication with the server device 40. The communication unit 24 is controlled by the control unit 25 to transmit the collected information or a content request to the server device 40. Further, the communication unit 24 receives content supplied from the server device 40.

The control unit 25 controls each unit in the terminal device 20, and controls each unit to operate depending to a user' operation. For example, the control unit 25 provides the collected information or makes a content request to the server device 40 depending to a user's operation. Further, the control unit 25 uses user identification information to provide the collected information or to make a content request so that the user who has provided the collected information or made a content request can be identified by the server device 40. Further, the control unit 25 periodically provides the collected information to the server device 40 if previously allowed by the user.

The control unit 25 reproduces content supplied from the server device 40 in response to a content request, and displays various items of information indicated by the content on the display unit 23. For example, when the content is information such as images or characters, the information is displayed on the display unit 23. For example, when the content indicates a URL (Uniform Resource Locator), the control unit 25 accesses a host indicated by the URL via the network 50 to obtain information, and displays the obtained information on the display unit 23. When terminating the reproduction of the content, the control unit 25 makes a content request to the server device 40. When the content supplied from the server device 40 is used, the control unit 25 notifies the server device 40 of the use of the content. For example, when an operation indicating that diet or exercise has been done based on the displayed content is performed by the user, the control unit 25 transmits a use notification indicating which content has been used to the server device 40. The control unit 25 may automatically determine which content has been used based on a detection result of the sensor unit 21. For example, when the user position is a shop position indicated by the content, the control unit 25 automatically determines that the content has been used, and transmits a use notification to the server device 40.

The life rhythm analyzing unit 30 has a collected information storing unit 31, an analysis processing unit 32 and an analysis results storing unit 33. The collected information storing unit 31 stores therein the collected information provided from the terminal device 20.

The analysis processing unit 32 analyzes a user's life rhythm pattern based on the collected information stored in the collected information storing unit 31. For example, the analysis processing unit 32 analyzes a pattern of daily life rhythm or weekly life rhythm and supplies an analysis result to the analysis storing unit 33.

The analysis storing unit 33 stores the life rhythm analysis result supplied from the analysis processing unit 32. The analysis results storing unit 33 supplies the stored analysis result to the content transmitting unit 45 in response to a request from the content transmitting unit 45.

The content registering unit 41 in the server device 40 registers content to be provided to the terminal device 20. The content is information associated with the actions configuring the user's life pattern. The content registering unit 41 registers the content in association with attributes of the actions. The content may be registered by a content provider online or may be registered offline based on an instruction from the content provider.

The content storing unit 42 stores therein the content registered in association with the attributes of the actions. The content stored in the content storing unit 42 is read by the content transmitting unit 45.

The environmental information acquiring unit 43 obtains environmental information on a user's life. The environmental information acquiring unit 43 obtains environmental information such as weather information including temperature, daylight hours, precipitation, wind direction and wind speed. In obtaining the weather information, the environmental information acquiring unit 43 obtains information corresponding to a user's position. The user's position may be previously registered in the server device 40 by the user, and the position detected by the location detecting unit 212 in the sensor unit 21 in the terminal device 20 may be used. Further, a user's future position is predicted and environmental information of an area including the predicted position may be obtained. The future position may be predicted with reference to a user's past action history, for example.

The environmental information storing unit 44 stores therein the environmental information obtained by the environmental information acquiring unit 43. The environmental information storing unit 44 is read by the content transmitting unit 45.

The content transmitting unit 45 determines a user who has made a content request, and reads the analysis result of the life rhythm of the determined user from the analysis results storing unit 33 in the life rhythm analyzing unit 30. The content transmitting unit 45 determines a user's next action based on the read analysis result, and reads environmental information corresponding to the determined action from the environmental information storing unit 44. Further, the content transmitting unit 45 reads content associated with the determined action and the read environmental information from the content storing unit 42, and transmits the content to a content request source. Further, the content transmitting unit 45 calculates a use rate indicating how often the content is used for content transmitting based on the content use notification from the terminal device 20. The calculated use rate may be stored in association with the content stored in the content storing unit 42, for example, or may be notified to the content registrant.

The communication unit 47 makes communication with the communication unit 24 in the terminal device 20, and receives a content request or the like supplied from the terminal device 20 and supplies it to the content transmitting unit 45. The communication unit 47 transmits the content supplied from the content transmitting unit 45 to the terminal device 20 in response to the content request.

There has been described the case in which the terminal device 20 uses the display unit 23 as a content presenting unit to display content. However, when the content is speech information, the content is presented by a speaker or the like. The content storing unit 42 and the environmental information storing unit 44 may be provided in an external device such as data center instead of the server device 40.

### <2. Operations of terminal device>

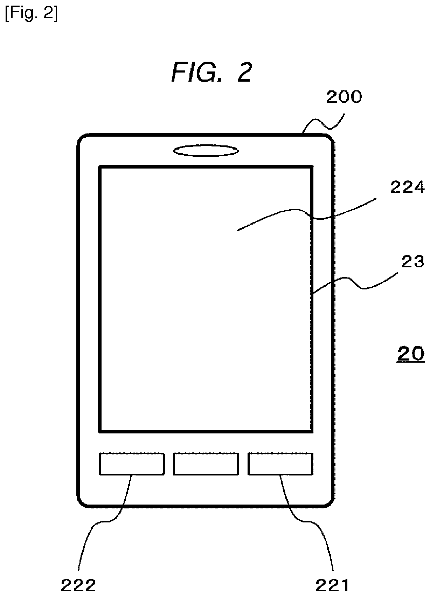

Fig. 2 exemplifies an outer profile of the terminal device 20. A housing 200 in the terminal device 20 is provided with operating switches 221 and 222 configuring the user interface unit 22. A touch panel 224 configuring the user interface unit 22 is provided on the display surface of the display unit 23. For example, the operating switch 221 is directed for reading an information input screen used for analyzing a life rhythm. The operating switch 222 is directed for reading a channel selection screen for designating a category of content to be obtained from the server device 40. The control unit 25 in the terminal device 20 displays the information input screen on the display unit 23 in response to an operation of the operating switch 221. Further, when the user operation is performed for displaying the information input screen, the control unit 25 determines which input operation the user has performed, based on the operation position of the touch panel 224 and the screen display, and performs an information input processing based on a discrimination result.

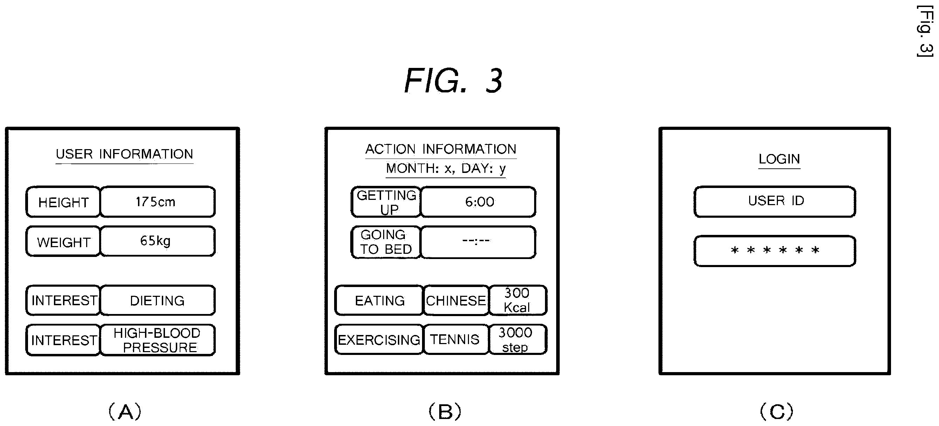

Fig. 3 exemplifies the information input screen. Fig. 3(A) exemplifies a user information input screen. Information for selecting user's physical features or user's highly-interested content can be input on the user information input screen. For example, in Fig. 3(A), there are provided text fields for inputting the physical features such as body height and body weight and text fields for inputting highly-interested items. However, the information inputting is not limited to the text fields. A spin button may be provided for inputting body height and body weight, or a list box or combo box may be provided for inputting highly-interested items.

Fig. 3(B) exemplifies an action information input screen. Action information which cannot be collected by the sensor unit 21 or action information by the user instead of the sensor unit 21 can be input on the action information input screen. For example, in Fig. 3(B), there are provided text fields for inputting wake-up time, bedtime, diet category or taken calories, exercise type and steps. However, the information inputting is not limited to the text fields as described above. A spin button may be provided for inputting wake-up time, bedtime and steps, and a list box or combo box may be provided for inputting diet category or exercise type. The action information is input in association with time information in order to identify when the action information relates to an action. For the time information to be associated, the time information indicating when the information input operation has been performed may be automatically generated in the control unit 25 or the user may input the time information.

Fig. 3(C) exemplifies a log-in screen. The log-in screen is provided with text fields for inputting a user ID for user identification.

The control unit 25 in the terminal device 20 displays the channel selection screen on the display unit 23 in response to an operation of the operating switch 222. Further, when the user operation has been performed for displaying the channel selection screen, the control unit 25 determines which channel selection operation the user has performed, based on the operation position of the touch panel 224 and the screen display.

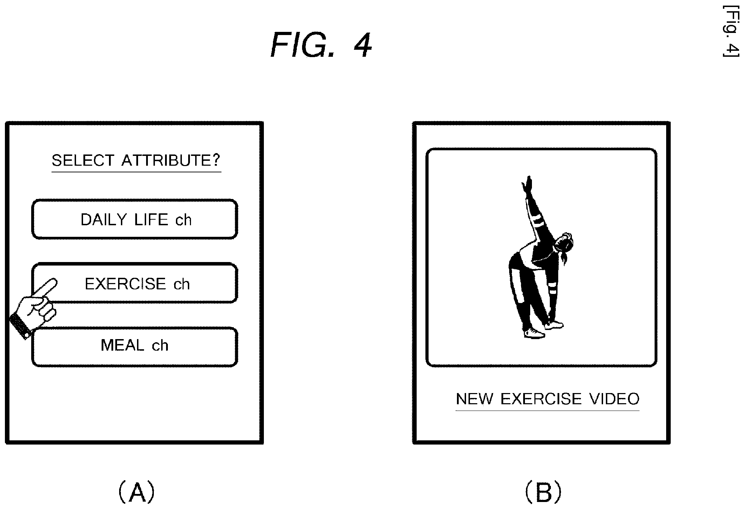

Fig. 4 exemplifies the channel selection screen. Any of multiple channels can be selected on the channel selection screen. For example, in Fig. 4(A), any of daily life channel, exercise channel and diet channel can be selected. The daily life channel is directed for timely providing information on diet, exercise and sleep corresponding to a user's life rhythm. The exercise channel is directed for timely providing exercise-related information or the like based on user's exercise history information in correspondence to the user's life rhythm. The diet channel is directed for timely providing diet-related information or the like corresponding to a user's preference in correspondence to the user's life rhythm. When the exercise channel is selected, content supplied from the server device 40 in correspondence to the user's life rhythm for exercise is displayed as shown in Fig. 4(B).

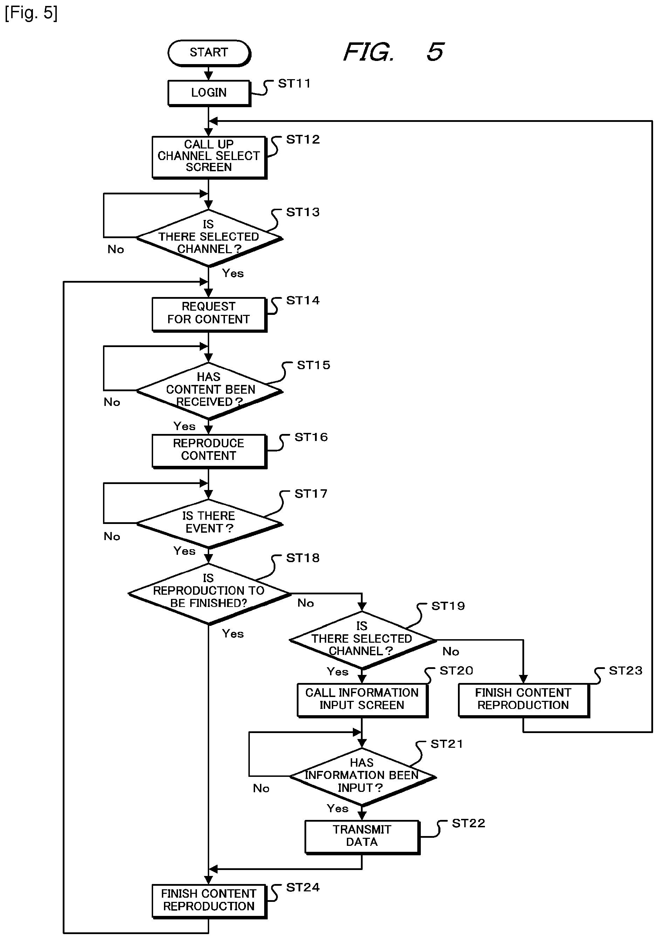

Fig. 5 is a flowchart showing operations of the terminal device. In step ST11, the control unit 25 logs in. The control unit 25 displays the log-in screen on the display unit 23 to receive user ID input. The control unit 25 transmits the input user ID to the server device 40, and when being notified of the completion of the user confirmation from the server device 40, proceeds to step ST12. When being notified of the fact that the user confirmation has not been made, the control unit 25 displays the fact on the display unit 23.

In step ST12, the control unit 25 calls the channel selection screen. The control unit 25 controls the display unit 23 to display the channel selection screen, and proceeds to step ST13.

In step ST13, the control unit 25 determines whether the channel selection has been made. When not detecting that the channel selection has been made, the control unit 25 returns to step ST13, and when detecting that the channel selection has been made, proceeds to step ST14.

In step ST14, the control unit 25 makes a content request. The control unit 25 requests content of the channel selected in step ST13 to the server device 40 and proceeds to step ST15.

In step ST15, the control unit 25 determines whether the content has been received. The control unit 25 returns to step ST15 until the content is supplied from the server device 40 in response to the content request, and proceeds to step ST16 when receiving the content transmitted from the server device 40.

In step ST16, the control unit 25 reproduces content. The control unit 25 reproduces the received content to display the information on the screen of the display unit 23, for example, and then proceeds to step ST17.

In step ST17, the control unit 25 determines whether an event has occurred. When an event has not occurred, for example, when a user operation has not been performed, the control unit 25 returns to step ST17, and when it is determined that the user operation has been performed, proceeds to step ST18.

In step ST18, the control unit 25 determines which of the reproduction end and the switch operation the event is. When the touch panel operation of the display unit 23 has been performed, for example, the control unit 25 determines that the event is the reproduction end, and proceeds to step ST24, and when the switch operation has been performed by any of the operating switches 221 and 222, proceeds to step ST19.

In step ST19, the control unit 25 determines whether the switch operation is the channel selection operation. When it is determined that the operating switch 222 reading the channel selection screen has been operated, the control unit 25 proceeds to step ST23. When it is determined that the operating switch 221 calling the information input screen has been operated, the control unit 25 proceeds to step ST20.

In step ST20, the control unit 25 calls the information input screen. The control unit 25 controls the display unit 23 to display the information input screen, and proceeds to step ST21.

In step ST21, the control unit 25 determines whether information has been input. When not detecting that information has been input, the control unit 25 returns to step ST21, and when detecting that information has been input, proceeds to step ST22.

In step ST22, the control unit 25 transmits data. The control unit 25 transmits the input information or the collected information to the server device 40, and proceeds to step ST24.

When proceeding from step ST19 to step ST23, the control unit 25 terminates the content reproduction. Since a new channel is to be selected, the control unit 25 terminates the content reproduction and returns to step ST12.

When proceeding from step ST18 or 22 to step ST24, the control unit 25 terminates the content reproduction and returns to step ST14.

When the reproduced content is used, for example, when the user operation indicating that the content is to be used is performed while the content is being reproduced, the control unit 25 notifies the server device 40 of the use of the content.

In this way, the terminal device 20 provides the collected information for analyzing a life rhythm to the server device 40. Thus, the collected information provided is used to analyze a life rhythm described later, thereby predicting user's future actions. Further, since user's future actions can be predicted, when the terminal device 20 makes a content request to the server device 40, content corresponding to the predicted action can be transmitted from the server device 40 to the terminal device 20. Therefore, content corresponding to the user's life rhythm can be previously provided. The collected information is notified from the terminal device 20 to the server device 40. Accordingly, content to be provided can be improved for increasing a use rate.

### <3. Operations of life rhythm analyzing unit>

The life rhythm analyzing unit 30 analyzes a user's life rhythm based on the collected information.

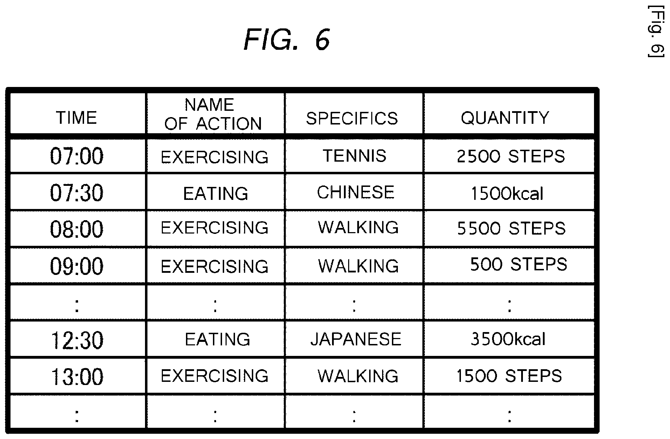

The collected information storing unit 31 stores the information input from the information input screen or the collected information obtained in the sensor unit 21 as a history per user. Fig. 6 exemplifies collected information stored in the collected information storing unit 31. For example, the collected information is stored in association with action name, type and quantity in time sequence. The "action name" classifies exercise and diet, and the "type" indicates an exercise type or diet type. Further, the "quantity" indicates the number of steps during exercise or the number of calories of food.

The analysis processing unit 32 analyzes a life rhythm by use of the collected information stored in the collected information storing unit 31. The analysis processing unit 32 analyzes an action pattern, an action-related quantity pattern such as exercise quantity pattern, and a preference pattern based on the collected information stored in the collected information storing unit 31.

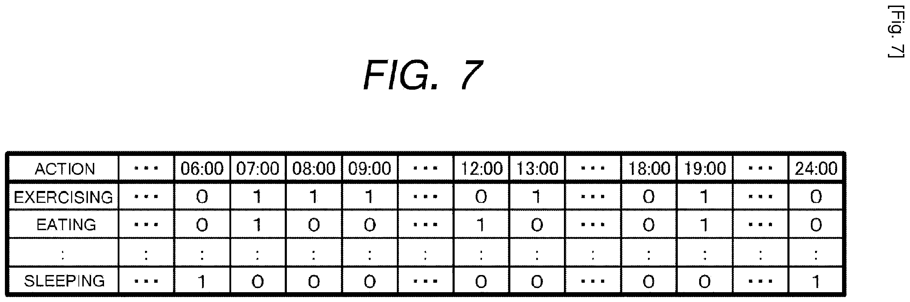

Fig. 7 exemplifies an action pattern analysis result. In the action pattern analysis result, which action is frequently done is indicated per action in time sequence. For example, the analysis processing unit 32 compares the stored information on exercise quantity or taken calories with preset thresholds, thereby analyzing whether exercise or diet has been done based on the comparison result. The analysis processing unit 32 sets the analysis result at "1" assuming that exercise is done when the exercise quantity is equal to or more than the threshold. Similarly, the analysis processing unit 32 sets the analysis result at" 1" assuming that diet is done when the number of taken calories is equal to or more than the threshold. When analyzing that neither exercise or diet has been done, the analysis processing unit 32 sets the analysis result at "0." The analysis processing unit 32 generates an action pattern analysis result assuming that the initial value of the action pattern analysis result is "0" and the daily analysis results at a set time are added to be frequency values. When the action pattern analysis result is generated in this way, the action pattern analysis result is a model related to the action pattern. Therefore, by use of the action pattern analysis result, it is possible to determine when and which action is often done, based on the frequency values of the action pattern analysis result.

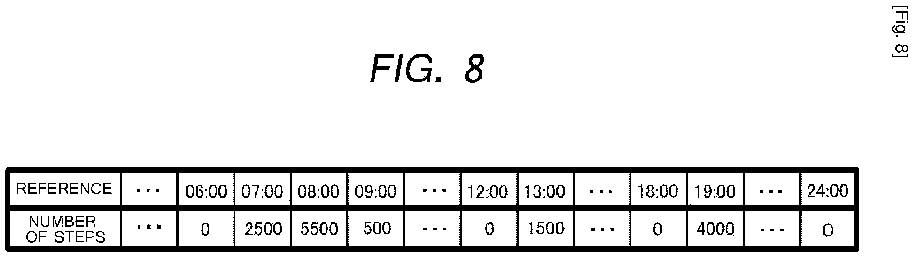

Fig. 8 exemplifies an exercise quantity pattern analysis result. The analysis processing unit 32 calculates a statistical value or average value of daily exercise quantities every hour, and assumes the exercise quantity pattern analysis result. When the exercise quantity pattern analysis result is generated in this way, the exercise quantity pattern analysis result is a model related to an expected value of the exercise quantity.

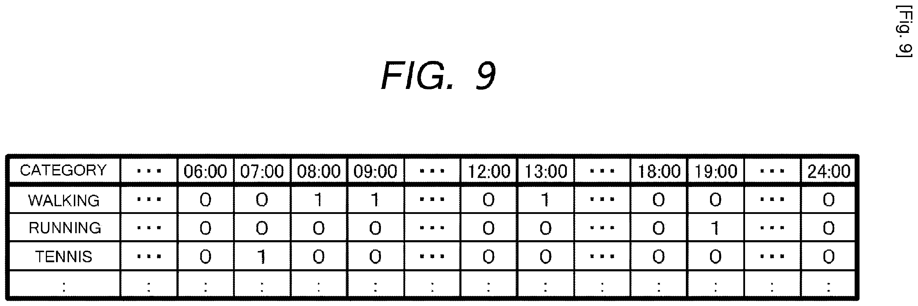

Fig. 9 exemplifies a preference pattern analysis result. Fig. 9 is a preference pattern analysis result of an exercise category. The preference pattern analysis result indicates which category of exercise is often done in time sequence. For example, the analysis processing unit 32 analyzes which category of exercise has been done based on the stored information on "type", and sets the analysis result of the category of the done exercise at "1." The analysis processing unit 32 sets the analysis result of the category of undone exercise at "0." The analysis processing unit 32 generates a preference pattern analysis result assuming that the initial value of the preference pattern analysis result is "0" similar to the action pattern analysis result and daily analysis results are added in time sequence to be frequency values. When the preference pattern analysis result is generated in this way, the preference pattern analysis result is a model on a preference of the exercise category. Thus, by use of the preference pattern analysis result, it is possible to analyze when and which category of exercise is often done, by the frequency values of the preference pattern analysis result.

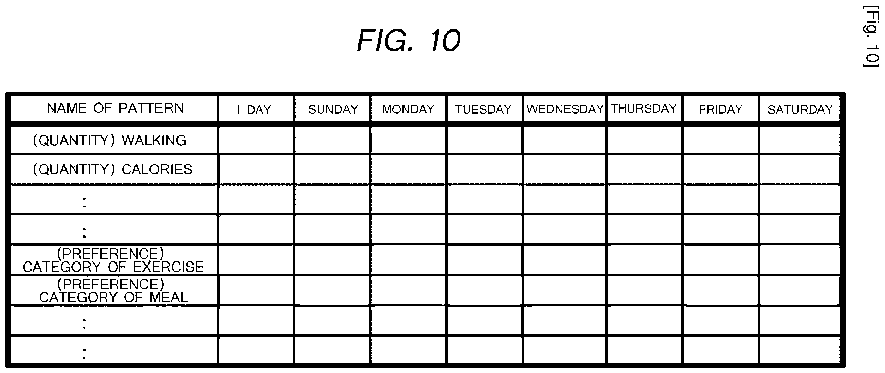

Since a life rhythm may be different between weekday and holiday, not only a daily life rhythm irrespective of day but also a day-based life rhythm may be analyzed. For the analysis of the day-based life rhythm, the collected information is divided per day, and the collected information of the day when the life rhythm is analyzed is used to analyze a life rhythm. Fig. 10 exemplifies a day-based pattern analysis result. The day-based pattern is configured of daily pattern analysis results irrespective of day and day-based pattern analysis results, that is, eight analysis result patterns. For example, the column of each day of (quantity) walking is associated with distribution data every hour in Fig. 8 corresponding to each date. The column of each day of (preference) exercise category is associated with distribution data every hour in Fig. 9 corresponding to each date. Similarly, a distribution of action patterns in Fig. 7 is also associated with each date and is managed in the table of Fig. 10.

The analysis results storing unit 33 stores therein the models on the life rhythm analyzed in the analysis processing unit 32, that is, the action pattern analysis result, the quantity pattern analysis result, the preference pattern analysis result and the like. When the collected information stored in the collected information storing unit 31 is updated and the life rhythm is analyzed by the analysis processing unit 32, the models of the life rhythm stored in the analysis results storing unit 33 are updated to new analysis results.

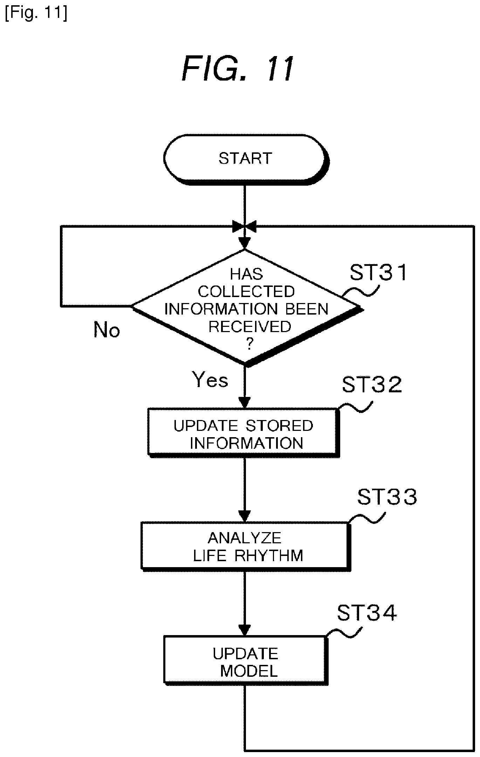

Fig. 11 is a flowchart showing operations of the life rhythm analyzing unit 30 when the life rhythm analyzing unit 30 is provided in the server device 40.

In step ST31, the life rhythm analyzing unit 30 determines whether the collected information has been received. When having been supplied with the information input from the information input screen or the information obtained by the sensor unit 21 from the terminal device 20 and having received the collected information, the life rhythm analyzing unit 30 proceeds to step ST32, and when having not received the collected information, returns to step ST31.

In step ST32, the life rhythm analyzing unit 30 updates the stored information. The collected information storing unit 31 in the life rhythm analyzing unit 30 stores the collected information newly supplied as a history, and proceeds to step ST33.

In step ST33, the life rhythm analyzing unit 30 analyzes a life rhythm. The collected information stored in the collected information storing unit 31 is updated so that the analysis processing unit 32 in the life rhythm analyzing unit 30 uses the updated information to analyze a life rhythm, and proceeds to step ST34. When analyzing the life rhythm by the day, the life rhythm analyzing unit 30 confirms of which day the collected information newly supplied is, and analyzes the life rhythm of the confirmed day.

In step ST34, the life rhythm analyzing unit 30 updates a model on the life rhythm. The analysis results storing unit 33 in the life rhythm analyzing unit 30 updates the stored model on the life rhythm based on the analysis result in step ST33, and returns to step ST31. When the life rhythm is analyzed by the day, the models of the same day are updated.

In this way, the life rhythm analyzing unit 30 analyzes the user's life rhythm based on the collected information, and generates an action pattern, a preference pattern and the like. Thus, the pattern analysis result is used thereby to predict user's future actions and the like.

### <4. Operations of server device>

The content information registered by the content registering unit 41 in the server device 40 is stored in the content storing unit 42.

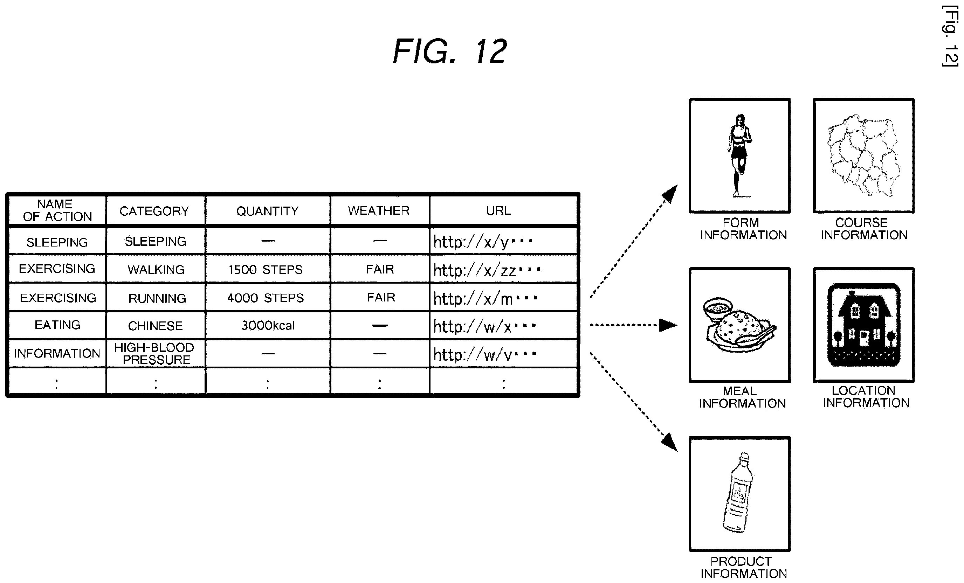

Fig. 12 exemplifies content information stored in the content storing unit 42. The content information contains content (such as URL (Uniform Resource Locator)) and metadata indicating attributes of the content. The metadata is information used for selecting content depending on a user's life rhythm or the like, and is registered by a content provider in association with the content attributes, for example. As the metadata, information on action name, action type and quantity used for analyzing a life rhythm, and environmental information are used.

The environmental information obtained by the environmental information acquiring unit 43 in the server device 40 is stored in the environmental information storing unit 44.

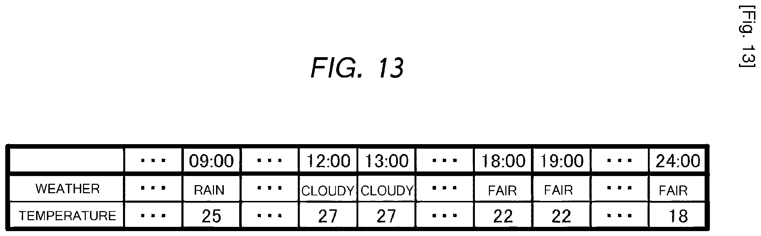

Fig. 13 exemplifies the environmental information stored in the environmental information storing unit 44. For the environmental information, information on weather or temperature is obtained as the environmental information. The environmental information is stored together with a time per divided area so as to obtain information depending on a content user's location. Fig. 13 exemplifies environmental information of one area.

The content transmitting unit 45 in the server device 40 determines parameters for searching a registered content for each time based on each model indicating the life rhythm, position information or channel information contained in a request from a client, and the stored environmental information. The content transmitting unit 45 selects content based on the determined parameters, and transmits the selected content to the client.

When a daily life channel is selected in the terminal device 20, the content transmitting unit 45 calculates an action probability in terms of a frequency at a content selecting time in the action pattern analysis result. Further, an action is determined by probability-based lot. When an individual channel such as diet or exercise is selected in the terminal device 20, the content transmitting unit 45 employs an action of the selected channel to select content, and thus does not draw probability-based lots.

The content selecting time is a time to be set for providing content related to an action to be done later with reference to a time when the content is requested. For example, the time when the content is requested is between 5 and 6 PM, the content selecting time is set between 6 and 7 PM that is one hour later. An interval between the time when the content is requested and the content selecting time is not limited to a preset fixed time, and may be changed depending on a time zone when the user-selected channel or content is requested. For example, in the time zone when an action changes in a short time from the life rhythm analysis result, an interval between the time when the content is requested and the content selecting time is shortened, thereby providing content according to the next action.

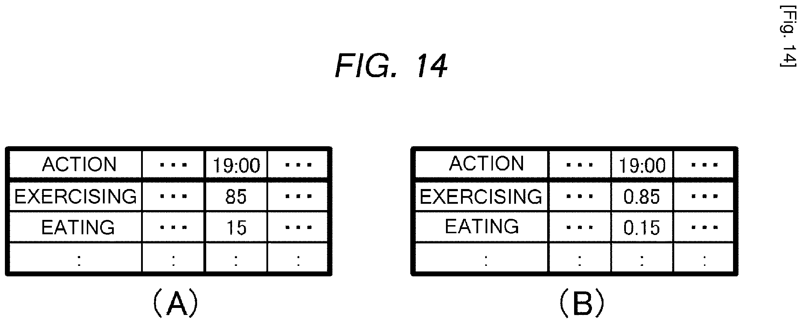

Fig. 14 is a diagram for explaining action determining operations. For example, as shown in Fig. 14(A), when the frequency value of exercise around 18 o'clock in the action pattern analysis result is "85" and the frequency value of diet is "15", the action probability is such that the probability of exercise is "0.85" and the probability of diet is "0.15" as shown in Fig. 14(B). Thus, an action is determined by the probability-based lot.

Then, the content transmitting unit 45 determines an item of content to be provided based on the preference pattern and determines a target standard of the value for selecting content to be provided based on the quantity pattern for the determined action.

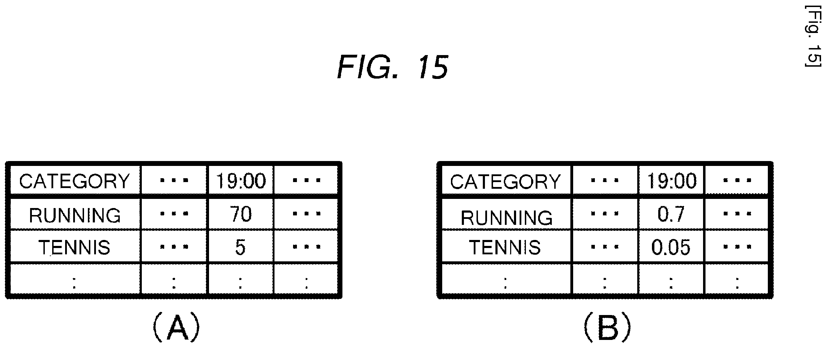

Fig. 15 is a diagram for explaining item determining operations when exercise is determined as an action. For example, as shown in Fig. 15(A), when the frequency value of running around 18 o'clock in the exercise preference pattern analysis result is "70" and the frequency value of tennis is "5", the item probability is such that the probability of running is "0.70" and the probability of tennis is "0.05" as shown in Fig. 15(B). Thus, an item is determined by the probability-based lot.

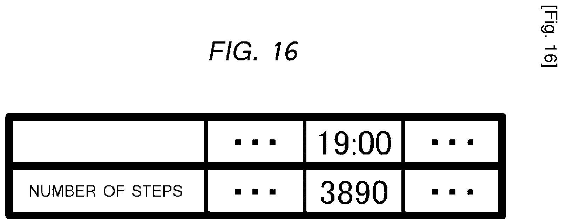

Fig. 16 is a diagram for explaining quantity target standard determining operations when exercise is determined as an action. For example, as shown in Fig. 16, when the exercise quantity between 6 to 7 PM in the quantity pattern analysis result is "3890", the target standard is determined at "3890 steps."

While Fig. 15 and Fig. 16 show the case in which exercise is determined as an action, the category of diet such as Chinese food or Japanese food is determined based on the preference pattern analysis result for diet. The number of taken calories is determined as a quantity reference value. Since the preference pattern for sleep has two kinds such as sleep and wake-up, a quantity parameter is not used.

The content transmitting unit 45 also performs an environmental information determination processing. For the determination of an environment, environmental information is determined based on a position indicated by the position information from the terminal device 20 and a content selecting time. For example, the environmental information at the content selecting time is selected from the environmental information of an area containing the position indicated by the position information. A position is not limited to the position indicated by the position information from the terminal device 20. For example, a position at the content selecting time may be estimated from the life rhythm with reference to the position indicated by the position information and the environmental information at the content selecting time may be selected from the environmental information of an area containing the estimated position.

As described above, the content transmitting unit 45 determines action, item, target standard and environmental information to be search parameters. For example, in the above example, the search parameters are action=exercise, action item=running, target standard=3890 steps, and environmental information=fine. Further, the content transmitting unit 45 selects content as transmitting candidates from the content stored in the content storing unit 42 based on the search parameters. For example, content having metadata such as action=exercise, action item=running and environmental information=fine is searched, and quantity meta of the searched content, which is close to target standard=3890 steps, is selected to be a transmitting candidate.

Furthermore, the content transmitting unit 45 randomly selects one content from a plurality of pieces of content selected as the transmitting candidates to be a transmitting content. The content transmitting unit 45 transmits the transmitting content to the terminal device 20. When one content is selected and transmitted based on the search parameters, the same content is repeatedly provided when a content request is made from the terminal device 20 multiple times in the same time zone. An operation of changing the search parameters, or the like is needed for providing a new content. However, the content transmitting unit 45 selects multiple pieces of content as transmitting candidates based on the search parameters. Further, the content transmitting unit 45 randomly selects and transmits content from the transmitting candidates, thereby providing a different content even when a content request is made from the terminal device multiple times in the same time zone.

The action may not correspond to any action item in the action pattern analysis result depending on a time. In this case, a user-input keyword is used as a search parameter for selecting transmitting candidates. Specifically, the keyword registered in the item of "interest" in the user information input screen is stored in the life rhythm analyzing unit 30 and content is selected with the keyword randomly selected from the stored keywords as a search parameter. For example, when the action does not correspond to any action item in the action pattern analysis result, the search parameter for action is set at "information." When the randomly-selected keyword is "high blood pressure", a plurality of pieces of content with the action of "information" and the type of "high blood pressure" are selected as the transmitting candidates from the registered content.

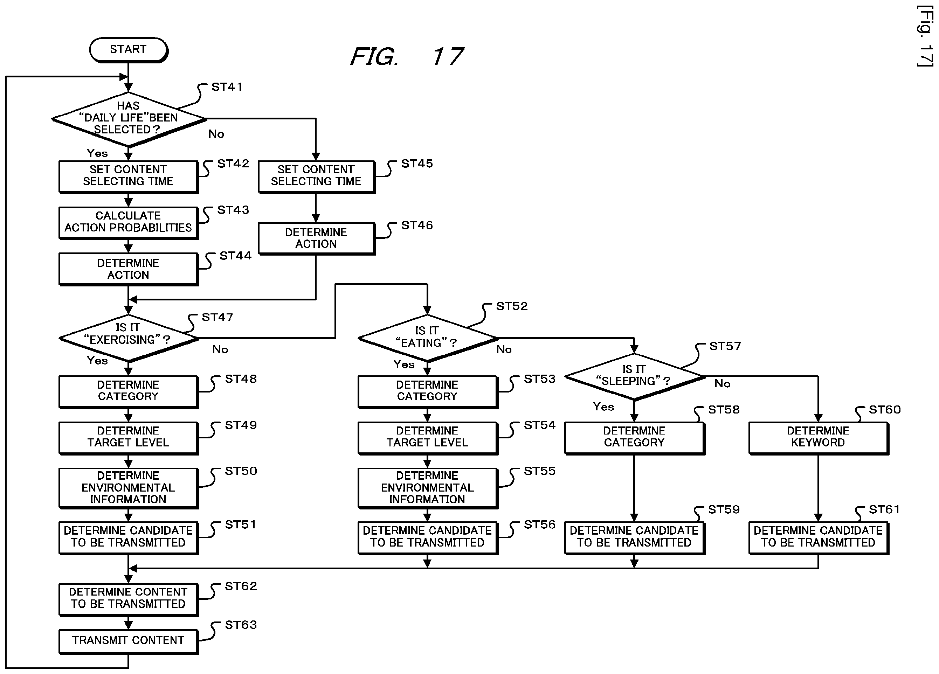

Fig. 17 is a flowchart showing operations of the content transmitting unit 45 when a content request is made. In step ST41, the content transmitting unit 45 determines whether the selected channel is daily life. When the selected channel is daily life, the content transmitting unit 45 proceeds to step ST42, and when the selected channel is other channel, proceeds to step ST45.

In step ST42, the content transmitting unit 45 sets a content selecting time. The content transmitting unit 45 sets a content selecting time for providing content related to a next action with reference to the time when the content request is made, and proceeds to step ST43.

In step ST43, the content transmitting unit 45 calculates an action probability. The content transmitting unit 45 calculates a probability per action based on the frequency value at the content selecting time in the action pattern analysis result, and proceeds to step ST44.

In step ST44, the content transmitting unit 45 determines an action. The content transmitting unit 45 draws action probability-based lots to determine an action, and proceeds to step ST47.

When proceeding from step ST41 to step ST45, the content transmitting unit 45 sets a content selecting time. The content transmitting unit 45 sets a content selecting time as in step ST42, and proceeds to step ST46.

In step ST46, the content transmitting unit 45 determines an action. The content transmitting unit 45 employs the action of the selected channel, and proceeds to step ST47.

In step ST47, the content transmitting unit 45 determines whether the determined action is exercise. When the determined action is exercise, the content transmitting unit 45 proceeds to step ST48, and when the determined action is not exercise, proceeds to step ST52.

In step ST48, the content transmitting unit 45 determines an item. The content transmitting unit 45 calculates a probability per item based on the frequency value at the content selecting time in the exercise preference pattern analysis result. Further, the content transmitting unit 45 draws item probability-based lots, and proceeds to step ST49.

In step ST49, the content transmitting unit 45 determines a target standard. The content transmitting unit 45 determines the exercise quantity at the content selecting time in the quantity pattern analysis result as the target standard, and proceeds to step ST50.

In step ST50, the content transmitting unit 45 determines environmental information. The content transmitting unit 45 selects information corresponding to the position indicated by the position information from the terminal device 20 and the content selecting time from the information stored in the environmental information storing unit 44 thereby to determine environmental information, and proceeds to step ST51.

In step ST51, the content transmitting unit 45 determines transmitting candidates. The content transmitting unit 45 selects content of the transmitting candidates from the content stored in the content storing unit 42 based on the search parameters with the action, the item and the like determined in steps ST44, 46 and 48 to 50 as the search parameters, and proceeds to step ST62.

When proceeding from step ST47 to step ST52, the content transmitting unit 45 determines whether the determined action is diet. When the determined action is diet, the content transmitting unit 45 proceeds to step ST53, and when the determined action is not diet, proceeds to step ST57.

In step ST53, the content transmitting unit 45 determines an item. The content transmitting unit 45 calculates a probability per category based on the frequency value at the content selecting time in the preference pattern analysis result for the category of cooking. Further, the content transmitting unit 45 draws lots based on category-based probability to determine an item, and proceeds to step ST54.

In step ST54, the content transmitting unit 45 determines a target standard. The content transmitting unit 45 determines a target standard based on the number of taken calories at the content selecting time in the quantity pattern analysis result, and proceeds to step ST55.

In step ST55, the content transmitting unit 45 determines environmental information. Similar to step ST50, the content transmitting unit 45 selects information corresponding to the position indicated by the position information from the terminal device 20 and the content selecting time from the information stored in the environmental information storing unit 44 thereby to determine environmental information, and proceeds to step ST56.

In step ST56, the content transmitting unit 45 determines transmitting candidates. The content transmitting unit 45 selects content of the transmitting candidates from the content stored in the content storing unit 42 based on the search parameters with the action, the item and the like determined in steps ST44, 46 and 53 to 55 as the search parameters, and proceeds to step ST62.

When proceeding from step ST52 to step ST57, the content transmitting unit 45 determines whether the determined action is sleep. When the determined action is sleep, the content transmitting unit 45 proceeds to step ST58, and when the determined action is not sleep, proceeds to step ST60.

In step ST58, the content transmitting unit 45 determines an item. The content transmitting unit 45 proceeds to step ST59 with the item as sleep.

In step ST59, the content transmitting unit 45 determines transmitting candidates. The content transmitting unit 45 selects content of the transmitting candidates from the content stored in the content storing unit 42 based on the search parameters with the action, the item and the like determined in steps ST44, 46 and 58 as the search parameters, and proceeds to step ST62.

When proceeding from step ST57 to step ST60, the content transmitting unit 45 determines a keyword. The content transmitting unit 45 randomly selects a keyword from the user-registered keywords, and proceeds to step ST61.

In step ST61, the content transmitting unit 45 determines transmitting candidates. The content transmitting unit 45 selects content of the transmitting candidates from the content stored in the content storing unit 42 based on the search parameters with the keyword determined in step ST60 as the search parameter, and proceeds to step ST62.

In step ST62, the content transmitting unit 45 determines a transmitting content. The content transmitting unit 45 randomly selects one content from a plurality of pieces of content selected as the transmitting candidates and assumes the content as a transmitting content, and proceeds to step ST63.

In step ST63, the content transmitting unit 45 transmits content. The content transmitting unit 45 transmits the transmitting content to the terminal device 20 having made a content request.

In this way, the content transmitting unit 45 determines the action, the item, the target standard, the environmental information and the like corresponding to the content selecting time as the search parameters from the user's life rhythm pattern analysis result analyzed in the life rhythm analyzing unit 30. The content transmitting unit 45 uses the search parameters to select the transmitting candidates of the content, and determines a transmitting content from the transmitting candidates to transmit the content to the terminal device 20. Thus, the content corresponding to the user's life rhythm can be previously provided. For example, information on food and restaurants can be provided before lunch time. Further, information on exercise can be provided before going back home. Furthermore, the environmental information is used to provide environment-based information such as information on eating by which a person feel cool when it is hot or information on exercise done indoor when it rains.

Further, the content transmitting unit 45 calculates a use rate indicating how often the content is used for the content transmitting, based on a content use notification from the terminal device 20. Thus, the calculated use rate is notified to the content registrant, thereby improving the content such that the use rate increases. The content use situation can be grasped per user based on the content use notification, and thus points or benefits are added depending on the use situation, for example, thereby enhancing the use rate.

As described above, with the information processing system 10, the client and server functions, the registered content, and each model indicating a life rhythm analyzed from each user's log are used simply to select a channel so that content having the channel attributes can be sequentially provided according to the rhythm such as time (when), quantity (how much) or preference (how).

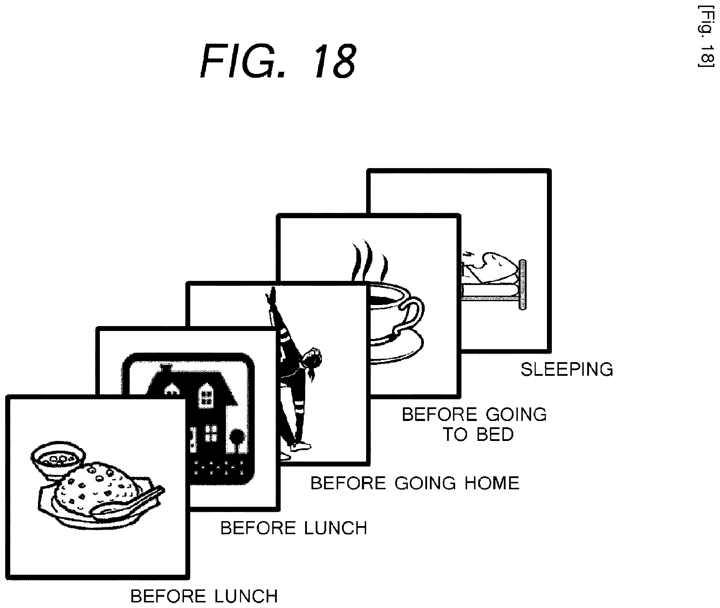

Therefore, the content user can timely obtain various pieces of content without progressively performing operations. For example, as shown in Fig. 18, there can be provided information on food and restaurants before lunch time, information on exercise before going back home, information on relaxation items before bedtime, and music or environmental sound for relaxation while sleeping. In this way, the content user can previously receive the content according to his/her life rhythm, and thus the content can be easily used. Further, content is selected in a combination of parameters such as category and quantity, and thus new content which the content user does not know can be provided. The content user only selects a channel indicating the attributes, thereby easily obtaining the personalized content according to his/her life rhythm. Furthermore, the content provider only sets metadata for content and registers the content, thereby efficiently providing information only to the user well-compatible with the content via the information processing system 10.

There has been described in the above form the case in which the life rhythm analyzing unit 30 is provided in the server device 40, but the life rhythm analyzing unit 30 may be provided in the terminal device 20. When the life rhythm analyzing unit 30 is provided in the server device 40, the terminal device 20 only provides the collected information to the server device 40 so that a life rhythm pattern is analyzed, and thus the processing of the terminal device 20 can be alleviated. When the life rhythm analyzing unit 30 is provided in the terminal device 20, the terminal device 20 contains the search parameters for selecting content according to a life rhythm at a content selecting time before the content request time in the content request instead of providing the collected information. The display unit 23 displays the content selected and supplied according to the life rhythm in the server device 40 based on the search parameters contained in the content request. Further, since the life rhythm pattern is analyzed in the terminal device 20, the user can confirm his/her life rhythm pattern by the terminal device 20.

A series of processing described in the specification may be executed in hardware, software, or in a combination structure thereof. When the processing is performed in software, a program recording a processing sequence therein is installed and executed in a memory in a computer incorporated in dedicated hardware. Alternatively, the program can be installed and executed in a general-purpose computer capable of executing various processing.

For example, the program may be previously recorded in a recording medium such as hard disc or ROM (Read Only Memory). Alternatively, the program may be temporarily or permanently stored (recorded) in a removable recording medium such as flexible disc, CD-ROM (Compact Disc Read Only Memory), MO (Magneto optical) disc, DVD (Digital Versatile Disc), magnetic disc or semiconductor memory card. Such a removable recording medium can be provided in so-called package software.

The program may be installed in a computer from a removable recording medium, or transferred to a computer in a wireless or wired manner via a network such as LAN (Local Area Network) or Internet from a download site. The computer can receive the program transferred in this way and install the program in an incorporated recording medium such as hard disc.

The present technique should not be interpreted only for the above technical embodiment. The technical embodiment discloses the present technique in examples, and it can be seen that those skilled in the art can modify or replace the embodiment without departing from the scope of the present technique. That is, Claims should be considered in order to understand the scope of the present technique.

### Industrial Applicability

With the terminal device, the external device, the information processing method, the program, and the information processing system according to the present technique, the terminal device collects information for selecting content according to a life rhythm, and provides the collected information and makes a content request to the external device. The external device presents content selected and supplied according to the life rhythm based on the collected information in response to the content request. The external device selects content based on the collected information for selecting content according to the life rhythm supplied from the terminal device in response to the content request from the terminal device, and transmits the selected content to the terminal device. Thus, the information corresponding to the user's life rhythm can be easily and timely used. For example, a health care content associated with diet or exercise can be timely used according to the user's life rhythm. A use frequency of a service of providing content can be enhanced and the service can be continuously used.

### Reference Signs List

10 Information processing system
20 Terminal device
21 Sensor unit
22 User interface unit
23 Display unit
24, 47 Communication unit
25 Control unit
30 Life rhythm analyzing unit
31 Collected information storing unit
32 Analysis processing unit
33 Analysis results storing unit
40 Server device
41 Content registering unit
42 Content storing unit
43 Environmental information acquiring unit
44 Environmental information storing unit
45 Content transmitting unit
50 Network
200 Housing
211 Movement sensor
212 Location detecting unit
221, 222 Operating switch
224 Touch panel

## Claims

1. An information processing system (10) comprising:
a user interface unit (22) that receives input from a user comprising the user's physical features;
a sensor unit (21) operable to collect information used for analyzing a life rhythm of a user;
a life rhythm analyzing unit (30) comprising a memory (31) that stores the information collected by the sensor unit and the information input from a user and is operable to analyze the user's life rhythm for individual days based on the collected information for each of the individual days stored in the memory, and operable to output an analysis result comprising a prediction of a next action of the user comprising at least one of a pattern of movement and a pattern of exercise corresponding to the user; and
a content transmitting unit (45) operable to acquire the analysis result of the life rhythm of the user from the life rhythm analyzing unit, and provide health-related advice information based on the predicted at least one of the pattern of movement and pattern of exercise corresponding to the user and based on the input from the user.

2. The information processing system of claim 1, wherein
the sensor unit is one selected from the list consisting of:
i. a movement sensor (211) that collects information corresponding to movement of the user,
ii. a pedometer (211) that collects information corresponding to a number of steps taken by the user, and
iii. a location detecting unit (212) that collects information corresponding to a location of the user.

3. The information processing system of claim 1, further comprising:
a content registering unit (41) that stores information associating each of a plurality of life rhythm analysis results and at least one piece of health-related advice information.

4. The information processing system of claim 3, wherein
the content transmitting unit acquires the health-related advice information to be provided based additionally on a comparison between the analysis result and the information stored in the content registering unit.

5. The information processing system of claim 1, further comprising:
an environmental information acquiring unit (43) that acquires environmental information corresponding to the user.

6. The information processing system of claim 5, wherein
the acquired environmental information includes at least one of weather information, daylight hours, precipitation, wind direction and wind speed.

7. The information processing system of claim 5, wherein
the content transmitting unit acquires the health-related advice information to be provided based additionally on the analysis result and the acquired environmental information corresponding to the user.

8. The information processing system of claim 1, further comprising:
a mobile terminal (20) that includes the sensor unit and the life rhythm analyzing unit; and
a server (40) that includes the content transmitting unit, wherein
the mobile terminal further includes an interface (22) that sends the analysis result to the server and receives the provided health-related advice information from the server.

9. The information processing system of claim 1, further comprising:
a mobile terminal that includes the sensor unit and an interface that sends the collected information to a server; and
the server that includes the life rhythm analyzing unit and the content transmitting unit and an interface that sends the provided health-related advice information to the mobile terminal.

10. An information processing method performed by an information processing system (10), the method comprising:
receiving (ST31) information used for analyzing a life rhythm of a user, the information having been collected by a sensor unit (21);
receiving, by a user interface unit (22), input from a user comprising the user's physical features; storing (ST32) the collected information for individual days and the information input from the user;
analyzing (ST33) the user's life rhythm based on the collected information stored for each of the individual days, and outputting (ST44,ST46) an analysis result comprising a prediction of a next action of the user comprising at least one of a pattern of movement and a pattern of exercise corresponding to the user;
acquiring the analysis result of the life rhythm based on the analyzing; and
providing (ST63) health-related advice information based on the predicted at least one of the pattern of movement and pattern of exercise corresponding to the user and based on the input from the user.

11. A computer program comprising code means adapted to perform all the steps of method claim 10, when said program is executed on a data processing system.

## Patentansprüche

1. Datenverarbeitungssystem (10), das Folgendes umfasst:
eine Anwenderschnittstelleneinheit (22), die eine Eingabe von einem Anwender empfängt, die die physischen Merkmale des Anwenders umfasst;
eine Sensoreinheit (21), die betrieben werden kann, um Informationen zu sammeln, die zum Analysieren eines Lebensrhythmus eines Anwenders verwendet werden;
eine Lebensrhythmus-Analyseeinheit (30), die einen Speicher (31) umfasst, der die Informationen, die durch die Sensoreinheit gesammelt werden, und die Informationen, die von einem Anwender eingegeben werden, speichert, und die betrieben werden kann, um den Lebensrhythmus des Anwenders für einzelne Tage auf der Basis der gesammelten Informationen für jeden der einzelnen Tage, die in dem Speicher gespeichert sind, zu analysieren, und die betrieben werden kann, um ein Analyseergebnis auszugeben, das eine Vorhersage einer nächsten Aktion des Anwenders umfasst, die ein Bewegungsmuster und/oder ein Übungsmuster in Übereinstimmung mit dem Anwender umfasst; und
eine Inhaltsübertragungseinheit (45), die betrieben werden kann, um das Analyseergebnis des Lebensrhythmus des Anwenders von der Lebensrhythmus-Analyseeinheit zu erfassen und gesundheitsbezogene Empfehlungsinformationen auf der Basis des vorhergesagten Bewegungsmusters und/oder Übungsmusters in Übereinstimmung mit dem Anwender und auf der Basis der Eingabe des Anwenders bereitzustellen.

2. Datenverarbeitungssystem nach Anspruch 1, wobei
die Sensoreinheit eine Einheit ist, die aus der Liste ausgewählt ist, die Folgendes enthält:
i. einen Bewegungssensor (211), der Informationen in Übereinstimmung mit einer Bewegung des Anwenders sammelt,
ii. einen Schrittzähler (211), der Informationen in Übereinstimmung mit einer Anzahl von Schritten, die durch den Anwender ausgeführt werden, sammelt, und
iii. eine Positionsdetektionseinheit (212), die Informationen in Übereinstimmung mit einer Position des Anwenders sammelt.

3. Datenverarbeitungssystem nach Anspruch 1, das ferner Folgendes umfasst:
eine Inhaltsregistrierungseinheit (41), die Informationen speichert, die jedes von mehreren Lebensrhythmus-Analyseergebnissen und wenigstens ein Element von gesundheitsbezogenen Empfehlungsinformationen verknüpfen.

4. Datenverarbeitungssystem nach Anspruch 3, wobei
die Inhaltsübertragungseinheit die gesundheitsbezogenen Empfehlungsinformationen, die bereitgestellt werden sollen, zusätzlich auf der Basis eines Vergleichs zwischen dem Analyseergebnis und den Informationen, die in der Inhaltsregistrierungseinheit gespeichert sind, erfasst.

5. Datenverarbeitungssystem nach Anspruch 1, das ferner Folgendes umfasst:
eine Umgebungsinformationen-Erfassungseinheit (43), die Umgebungsinformationen in Übereinstimmung mit dem Anwender erfasst.

6. Datenverarbeitungssystem nach Anspruch 5, wobei
die erfassten Umgebungsinformationen Wetterinformationen, Tageslichtstunden, Niederschläge, Windrichtung und/oder Windgeschwindigkeit umfassen.

7. Datenverarbeitungssystem nach Anspruch 5, wobei
die Inhaltsübertragungseinheit die gesundheitsbezogenen Empfehlungsinformationen, die bereitgestellt werden sollen, zusätzlich auf der Basis des Analyseergebnisses und der erfassten Umgebungsinformationen in Übereinstimmung mit dem Anwender erfasst.

8. Datenverarbeitungssystem nach Anspruch 1, das ferner Folgendes umfasst:
ein mobiles Endgerät (20), das die Sensoreinheit und die Lebensrhythmus-Analyseeinheit umfasst; und
einen Server (40), der die Inhaltsübertragungseinheit umfasst, wobei
das mobile Endgerät ferner eine Schnittstelle (22) umfasst, die das Analyseergebnis an den Server sendet und die bereitgestellten gesundheitsbezogenen Empfehlungsinformationen von dem Server empfängt.

9. Datenverarbeitungssystem nach Anspruch 1, das ferner Folgendes umfasst:
ein mobiles Endgerät, das die Sensoreinheit und eine Schnittstelle, die die gesammelten Informationen an einen Server sendet, umfasst; und
den Server, der die Lebensrhythmus-Analyseeinheit und die Inhaltsübertragungseinheit und eine Schnittstelle, die die bereitgestellten gesundheitsbezogenen Empfehlungsinformationen an das mobile Endgerät sendet, umfasst.

10. Datenverarbeitungsverfahren, das durch ein Datenverarbeitungssystem (10) durchgeführt wird, wobei das Verfahren die folgenden Schritte umfasst:
Erhalten (ST31) von Informationen, die zum Analysieren eines Lebensrhythmus eines Anwenders verwendet werden, wobei die Informationen durch eine Sensoreinheit (21) gesammelt worden sind;
Erhalten durch eine Anwenderschnittstelle (22) einer Eingabe von einem Anwender, die die physischen Merkmale des Anwenders umfasst;
Speichern (ST32) der gesammelten Informationen für einzelne Tage und der Informationen, die durch den Anwender eingegeben wurden;
Analysieren (ST33) des Lebensrhythmus des Anwenders auf der Basis der gesammelten Informationen, die für jeden der einzelnen Tage gespeichert sind, und Ausgeben (ST44, ST46) eines Analyseergebnisses, das eine Vorhersage einer nächsten Aktion des Anwenders umfasst, die ein Bewegungsmuster und/oder ein Übungsmuster in Übereinstimmung mit dem Anwender umfasst;
Erfassen des Analyseergebnisses des Lebensrhythmus auf der Basis der Analyse; und
Bereitstellen (ST63) von gesundheitsbezogenen Empfehlungsinformationen auf der Basis des vorhergesagten Bewegungsmusters und/oder Übungsmusters in Übereinstimmung mit dem Anwender und auf der Basis der Eingabe durch den Anwender.

11. Computerprogramm, das Codemittel umfasst, die ausgelegt sind, alle Schritte des Verfahrens nach Anspruch 10 auszuführen, wenn das Programm auf einem Datenverarbeitungssystem ausgeführt wird.

## Revendications

1. Système (10) de traitement d'informations, comprenant :
une unité d'interface utilisateur (22) qui reçoit une entrée en provenance d'un utilisateur, comprenant les caractéristiques physiques de l'utilisateur ;
une unité capteur (21) utilisable pour collecter des informations servant à l'analyse d'un rythme de vie d'un utilisateur ;
une unité d'analyse de rythme de vie (30) comprenant une mémoire (31) qui stocke les informations collectées par l'unité capteur et l'entrée d'informations en provenance d'un utilisateur, et est utilisable pour analyser le rythme de vie de l'utilisateur pour des jours individuels sur la base des informations collectées pour chacun des jours individuels stockés dans la mémoire, et utilisable pour délivrer en sortie un résultat d'analyse comprenant une prédiction d'une action à venir de l'utilisateur comprenant au moins un schéma parmi un schéma de mouvements et un schéma d'exercices correspondant à l'utilisateur ; et
une unité de transmission de contenu (45) utilisable pour acquérir le résultat d'analyse du rythme de vie de l'utilisateur auprès de l'unité d'analyse de rythme de vie, et fournir des informations de conseil sur la santé sur la base de l'au moins un schéma prédit parmi le schéma de mouvements et le schéma d'exercices correspondant à l'utilisateur et sur la base de l'entrée en provenance de l'utilisateur.

2. Système de traitement d'informations selon la revendication 1, dans lequel
l'unité capteur consiste en un élément sélectionné dans la liste constituée par :
i. un capteur de mouvement (211) qui collecte des informations correspondant à un mouvement de l'utilisateur,
ii. un podomètre (211) qui collecte des informations correspondant à un nombre de pas effectués par l'utilisateur, et
iii. une unité de détection de localisation (212) qui collecte des informations correspondant à une localisation de l'utilisateur.

3. Système de traitement d'informations selon la revendication 1, comprenant en outre :
une unité d'enregistrement de contenu (41) qui stocke des informations associant chacun d'une pluralité de résultats d'analyse de rythme de vie et au moins un élément d'informations de conseil sur la santé.

4. Système de traitement d'informations selon la revendication 3, dans lequel
l'unité de transmission de contenu acquiert les informations de conseil sur la santé à fournir sur la base en outre d'une comparaison entre le résultat d'analyse et les informations stockées dans l'unité d'enregistrement de contenu.

5. Système de traitement d'informations selon la revendication 1, comprenant en outre :
une unité d'acquisition d'informations ambiantes (43) qui acquiert des informations ambiantes correspondant à l'utilisateur.

6. Système de traitement d'informations selon la revendication 5, dans lequel
les informations ambiantes acquises comportent au moins un élément dans le groupe constitué par des informations météorologiques, des heures de clarté, une précipitation, une direction du vent et une vitesse du vent.

7. Système de traitement d'informations selon la revendication 5, dans lequel
l'unité de transmission de contenu acquiert les informations de conseil sur la santé à fournir sur la base en outre du résultat d'analyse et des informations ambiantes acquises correspondant à l'utilisateur.

8. Système de traitement d'informations selon la revendication 1, comprenant en outre :
un terminal mobile (20) qui comporte l'unité capteur et l'unité d'analyse de rythme de vie ; et
un serveur (40) qui comporte l'unité de transmission de contenu,
le terminal mobile comportant en outre une interface (22) qui envoie le résultat d'analyse au serveur et reçoit les informations de conseil sur la santé fournies en provenance du serveur.

9. Système de traitement d'informations selon la revendication 1, comprenant en outre :
un terminal mobile qui comporte l'unité capteur et une interface qui envoie les informations collectées à un serveur ; et
le serveur qui comporte l'unité d'analyse de rythme de vie et l'unité de transmission de contenu et une interface qui envoie les informations de conseil sur la santé fournies au terminal mobile.

10. Procédé de traitement d'informations mis en œuvre par un système (10) de traitement d'informations, le procédé comprenant :
la réception (ST31) d'informations servant à l'analyse d'un rythme de vie d'un utilisateur, les informations ayant été collectées par une unité capteur (21) ;
la réception, par une unité d'interface utilisateur (22), d'une entrée en provenance d'un utilisateur, comprenant les caractéristiques physiques de l'utilisateur ;
le stockage (ST32) des informations collectées pour des jours individuels et de l'entrée d'informations en provenance de l'utilisateur ;
l'analyse (ST33) du rythme de vie de l'utilisateur sur la base des informations collectées stockées pour chacun des jours individuels, et
la délivrance en sortie (ST44, ST46) d'un résultat d'analyse comprenant une prédiction d'une action à venir de l'utilisateur comprenant au moins un schéma parmi un schéma de mouvements et un schéma d'exercices correspondant à l'utilisateur ;
l'acquisition du résultat d'analyse du rythme de vie sur la base de l'analyse ; et
la fourniture (ST63) d'informations de conseil sur la santé sur la base de l'au moins un schéma prédit parmi le schéma de mouvements et le schéma d'exercices correspondant à l'utilisateur et sur la base de l'entrée en provenance de l'utilisateur.

11. Programme d'ordinateur, comprenant des moyens formant code adaptés à mettre en œuvre toutes les étapes de procédé selon la revendication 10, lorsque ledit programme est exécuté sur un système de traitement de données.
